(19) Europäisches Patentamt
European Patent Office
Office européen des brevets

(11) EP 0 631 183 B1

(12) **EUROPÄISCHE PATENTSCHRIFT**

(45) Veröffentlichungstag und Bekanntmachung des Hinweises auf die Patenterteilung:
**09.10.1996 Patentblatt 1996/41**

(51) Int Cl.6: **G03C 7/392**, G03C 7/396, C08G 61/02, C07C 39/17

(21) Anmeldenummer: **94108787.6**

(22) Anmeldetag: **08.06.1994**

(54) **Farbfotografisches Aufzeichnungsmaterial**

Color photographic recording material

Matériau de reproduction photographique couleur

(84) Benannte Vertragsstaaten:
**DE FR GB NL**

(30) Priorität: **21.06.1993 DE 4320445**

(43) Veröffentlichungstag der Anmeldung:
**28.12.1994 Patentblatt 1994/52**

(73) Patentinhaber: **Agfa-Gevaert AG**
**D-51373 Leverkusen (DE)**

(72) Erfinder:
- **Weber, Beate, Dr.**
  **D-42799 Leichlingen (DE)**
- **Hagemann, Jörg, Dr.**
  **D-51065 Köln (DE)**
- **Helling, Günter, Dr.**
  **D-51519 Odenthal (DE)**
- **Geiger, Markus, Dr.**
  **D-40764 Langenfeld (DE)**

(56) Entgegenhaltungen:
**EP-A- 0 486 216** **EP-A- 0 508 398**
**FR-A- 1 399 044**

- **PATENT ABSTRACTS OF JAPAN vol. 13, no. 389 (P-925) (3737) 29. August 1989 & JP-A-01 137 258 (FUJI PHOTO FILM) 25. November 1987**
- **PATENT ABSTRACTS OF JAPAN vol. 13, no. 389 (P-925) (3737) 29. August 1989 & JP-A-01 137 258**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 631 183 B1

## Beschreibung

Die Erfindung betrifft ein farbfotografisches Aufzeichnungsmaterial mit einer verbesserten Farbstabilität.

Farbfotografische Materialien enthalten üblicherweise wenigstens einen Gelbkuppler, wenigstens einen Purpurkuppler und wenigstens einen Blaugrünkuppler, aus denen durch Belichtung und Entwicklung die entsprechenden Farbstoffe entstehen. Diese Farbstoffe, insbesondere die Farbstoffe, die dem Licht ständig ausgesetzt sind, sollen eine hohe Farbstabilität aufweisen, wobei besonderer Wert darauf gelegt wird, daß die Farbstabilität von allen drei Farben möglichst gleich gut ist, damit bei einem geringfügigen Verblassen keine Farbverfälschung eintritt.

Insbesondere gelbe Farbstoffe, die aus Kupplern mit einer offenkettigen Ketomethylengruppierung erzeugt werden, bedürfen sowohl der Stabilisierung gegen Licht als auch gegen das Verblassen im Dunkeln (dark-fading).

In US 3 700 455 wird bereits der Vorschlag gemacht, diese Aufgabe mit Bisphenolverbindungen als Stabilisatoren zu lösen. Der damit erzielte Effekt ist allerdings noch nicht ausreichend.

Dreikernphenole, bei denen die Phenolreste über Alkylgruppen miteinander verknüpft sind, werden zum gleichen Zweck in JP-A-11 37 258 und EP-A-508 398 vorgeschlagen. Auch deren Wirkung ist noch nicht ausreichend.

Es wurde jetzt überraschend gefunden, daß ganz bestimmte mehrkernige Phenole diese Aufgabe weit besser lösen können.

Gegenstand der Erfindung ist daher ein farbfotografisches Material, das auf einem Träger wenigstens eine blauempfindliche, wenigstens einen Gelbkuppler enthaltende Silberhalogenidemulsionsschicht, wenigstens eine grünempfindliche, wenigstens einen Purpurkuppler enthaltende Silberhalogenidemulsionsschicht, wenigstens eine rotempfindliche, wenigstens einen Blaugrünkuppler enthaltende Silberhalogenidemulsionsschicht sowie übliche nicht-lichtempfindliche Schichten enthält, dadurch gekennzeichnet, daß wenigstens eine blauempfindliche, einen Gelbkuppler enthaltende Schicht eine Verbindung der Formel (I)

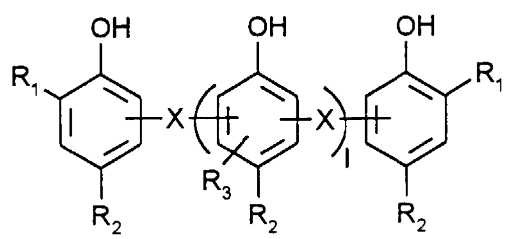

(I)

enthält, worin

$R_1$ Wasserstoff oder Alkyl,

$R_2$ Wasserstoff oder Alkyl,

$R_3$ Wasserstoff oder Alkyl,

1 1 bis 20, vorzugsweise 2 bis 8,

X Cycloalkylen, vorzugsweise

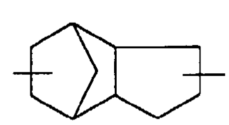

bedeuten.

Die Alkylgruppen $R_1$, $R_2$ und $R_3$ können geradkettig, verzweigt oder cyclisch sowie unsubstituiert oder substituiert sein. Vorzugsweise besitzen sie 1 bis 12 C-Atome und sind nicht weiter substituiert. Die Reste $R_1$ können untereinander gleich oder verschieden sein. Gleiches gilt für die Reste $R_2$.

Vorzugsweise ist $R_3$ Wasserstoff und sind alle Reste $R_2$ Alkyl. X ist bevorzugt

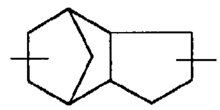

und $R_2$ tert.-Butyl.

Geeignete Verbindungen der Formel (I) sind:

I-1: $R_1 = C(CH_3)_3$, $R_2 = CH_3$, $R_3 = H$

X = 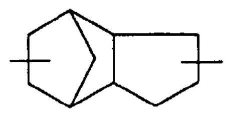

$\overline{M}_w = 1400$ I-2: $R_1 = H$, $R_2 = CH(CH_3)_2$, $R_3 = H$, X =

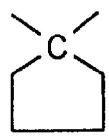

$\overline{M}_w \sim 1200$

Die Verbindungen der Formel I werden der wenigstens einer blauempfindlichen, wenigstens einen Gelbkuppler enthaltenden Silberhalogenidemulsionsschicht insbesondere in einer Menge von 0,1 bis 2 Mol/Mol Kuppler zugesetzt. Bei den Gelbkupplern handelt es sich vorzugsweise um 2-Äquivalent-pivaloylgelbkuppler, deren Abgangsgruppe entweder über Sauerstoff oder über Stickstoff mit der kuppelnden Position des Kupplers verbunden ist. Die Gelbkuppler werden vorzugsweise in einer Menge von 0,1 bis 1 mmol/$m^2$ fotografisches Material eingesetzt.

Geeignete Gelbkuppler sind:

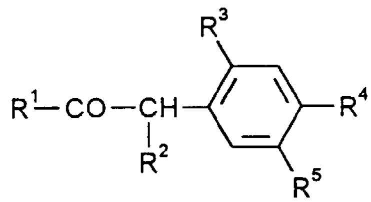

Y-1:

$R^1 = -C_4H_9$-t:

$R^2$ = 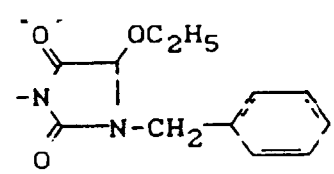 ;

$R^3 = Cl$; $R^4 = H$;

$R^5$ = 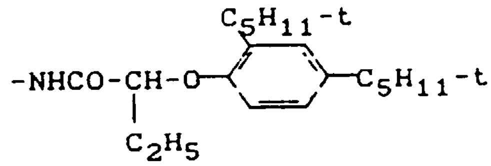

Y-2:
$R^1$ = $-C_4H_9$-t;

$R^2$ = 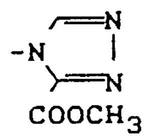 ;

$R^3$ = $-OC_{16}H_{33}$; $R^4$ = H; $R^5$ = $-SO_2NHCH_3$
Y-3:
$R^1$ = $-C_4H_9$-t;

$R^2$ = 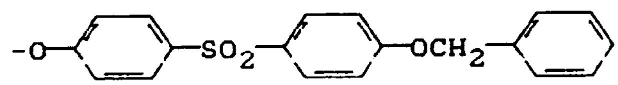 ;

$R^3$ = Cl
$R^4$ = H; $R^5$ = $-NHSO_2-C_{16}H_{33}$
Y-4: $R^1$ = $-C_4H_9$-t;

$R^2$ = 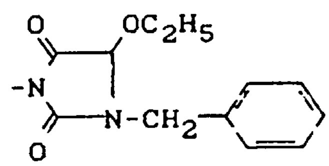 ;

$R^3$ = Cl;
$R^4$ = H; $R^5$ = $-COOC_{12}H_{25}$
Y-5:
$R^1$ = $-C_4H_9$-t:

$R^2$ = 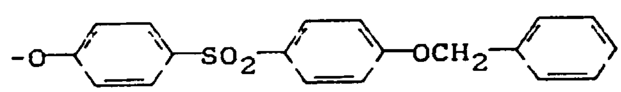 ;

$R^3$ = Cl;
$R^4$ = H;

$R^5$ = 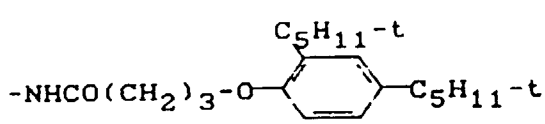

Y-6:

$R^1$ = $-C_4H_9$-t;

$R^2$ = 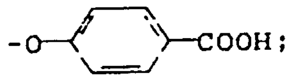 ; $R^3$ = Cl; $R^4$ = H;

$R^5$ = 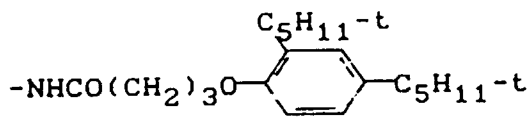

Y-7:
$R^1$ = $-C_4H_9$-t;

$R^2$ = 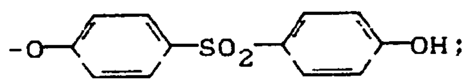 ; $R^3$ = Cl;

$R^4$ = H; $R^5$ = $-NHSO_2-C_{16}H_{33}$
Y-8:
$R^1$ = $-C_4H_9$-t;

$R^2$ = 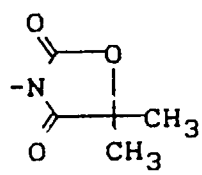 ;

$R^3$ = Cl; $R^4$ = H;

$R^5$ = 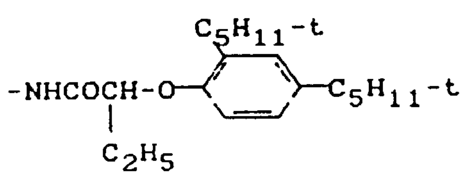

Y-9:
$R^1$ = $-C_4H_9$-t:

$R^2$ = 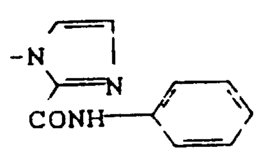 ;

$R^3$ = $-OC_{16}H_{33}$;
$R^4$ = H; $R^5$ = $-SO_2NHCOC_2H_5$
Y-10:
$R^1$ = $-C_4H_9$-t;

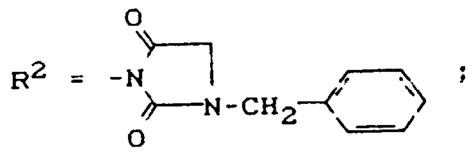

$R^3$ = Cl; $R^4$ = H

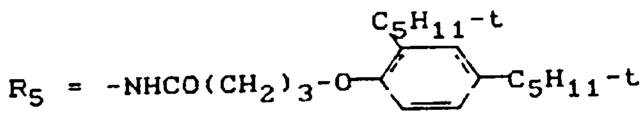

Y-11:
$R^1$ = $-C_4H_9$-t;

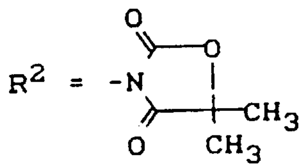 ; $R^3$ = Cl; $R^4$ = H;

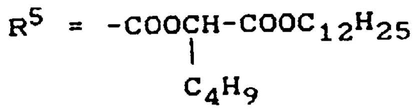

Y-12:
$R^1$ = $-C_4H_9$-t;

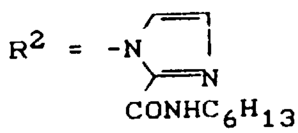

; $R^3$ = Cl; $R^4$ = H;

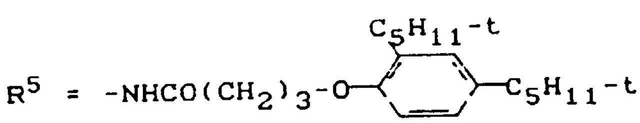

Y-13:
$R^1$ = $-C_4H_9$-t:

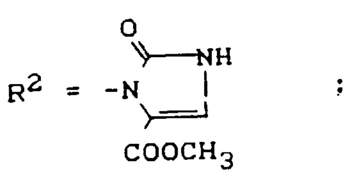

$R^3$ = $-OC_{16}H_{33}$; $R^4$ = H;
$R^5$ = $-SO_2NHCH_3$

Y-14:
$R^1$ = $-C_4H_9$-t;

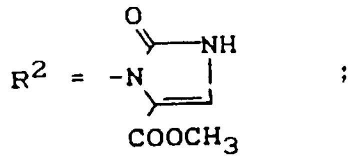

$R^3$ = Cl; $R^4$ = H;

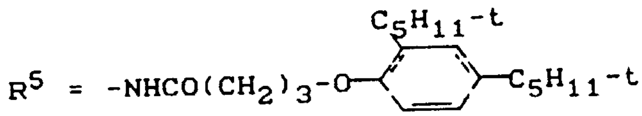

Y-15:

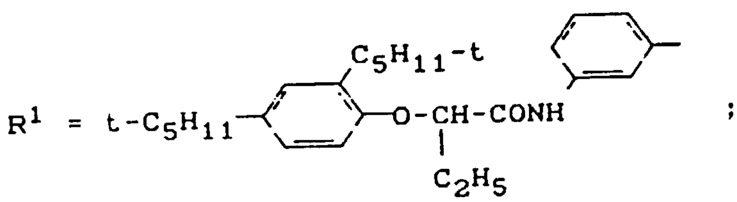

$R^2$, $R^4$, $R^5$ = H; $R^3$ = $-OCH_3$
Y-16:

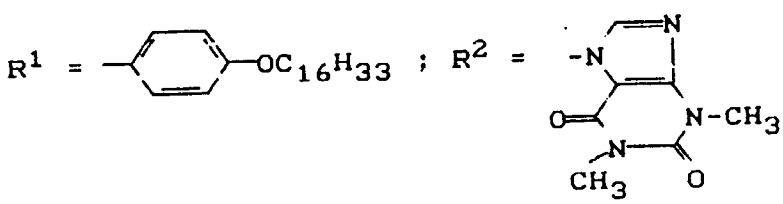

$R^3$, $R^5$ = $-OCH_3$; $R^4$ = H
Y-17:

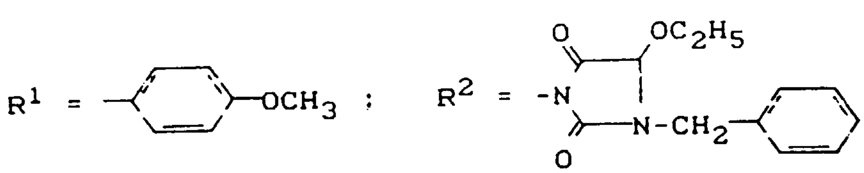

$R^3$ = Cl; $R^4$ = H; $R^5$ = $-COOC_{12}H_{25}$
Y-18:

$R^1$ = 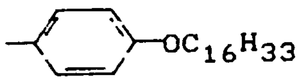 ; $R^2$ = 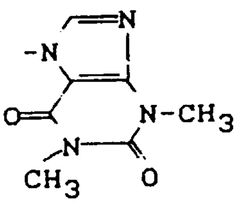

$R^3$ = Cl; $R^4$, $R^5$ = $-OCH_3$

Y-19:

$R^1$ = 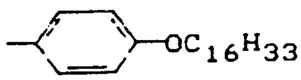 ; $R^2$ = 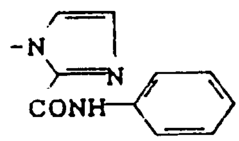

$R^3$ = $-OCH_3$; $R^4$ = H; $R^5$ = $-SO_2N(CH_3)_2$

Y-20:

$R^1$ = 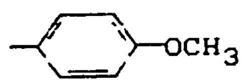 ;

$R^2$ = 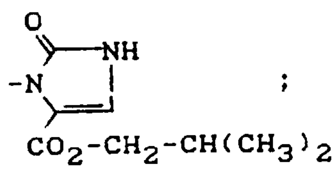 ;

$R^3$ = $-OCH_3$; $R^4$ = H;

$R_5$ = 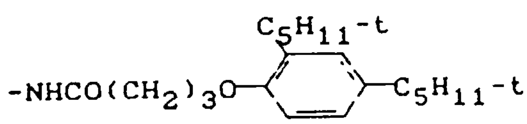

Y-21 $R_1$ = t-$C_4H_9$

$R_2$ = 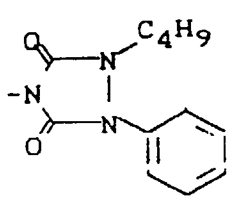

$R_3$ = $OCH_3$, $R_4$ = H, $R_5$ = $NHCOCH(CH_3)$ $CH_2$-$SO_2$-$C_{12}H_{25}$

Y-22:

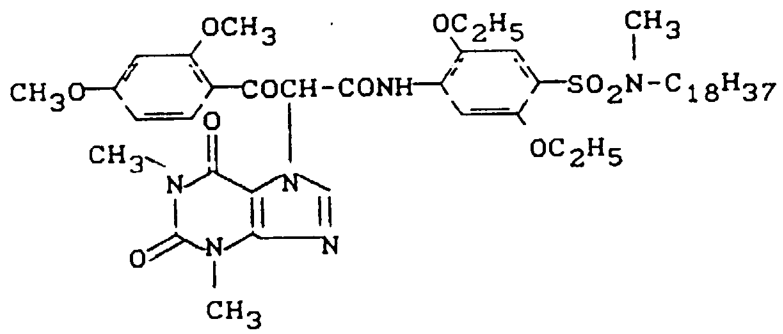

Y-23

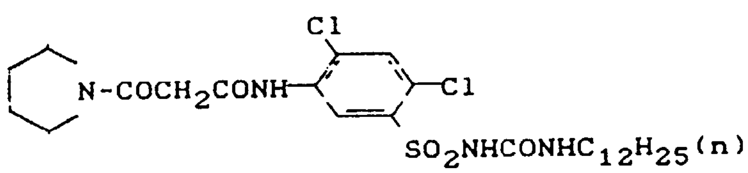

Y-24

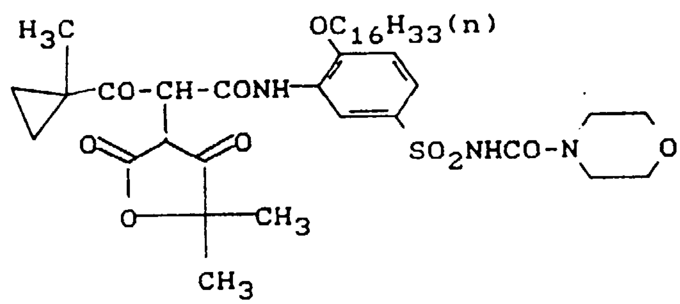

Y-25

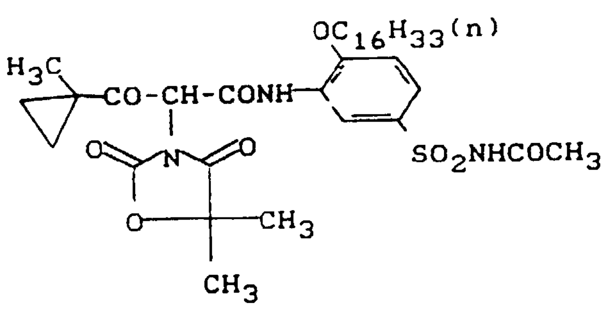

Y-26

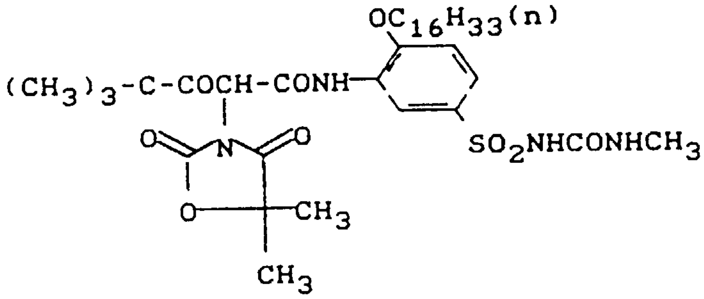

Der Träger kann reflektierend oder transparent sein.

Als Silberhalogenide der farbkupplerhaltigen und der farbkupplerfreien Silberhalogenidemulsionsschichten kommen AgBr, AgBrCl, AgBrCll und AgCl in Betracht.

Vorzugsweise enthalten die Silberhalogenide aller lichtempfindlichen Schichten einschließlich der erfindungsgemäßen Zwischenschichten wenigstens 80 Mol-% Chlorid, insbesondere 95 bis 100 Mol-% Chlorid, 0 bis 5 Mol-% Bromid und 0 bis 1 Mol-% Iodid. Die Silberhalogenidemulsionen können direkt positiv arbeitende oder vorzugsweise negativ arbeitende Emulsionen sein.

Bei dem Silberhalogenid kann es sich um überwiegend kompakte Kristalle handeln, die z.B. regulär kubisch oder oktaedrisch sind ober Übergangsformen aufweisen können. Vorzugsweise können aber auch verzwillingte, z.B. plättchenförmige Kristalle vorliegen, deren durchschnittliches Verhältnis von Durchmesser zu Dicke bevorzugt wenigstens 5:1 ist, wobei der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke größer als 5:1 ist, z.B. 12:1 bis 30:1.

Die Silberhalogenidkörner können auch einen mehrfach geschichteten Kornaufbau aufweisen, im einfachsten Fall mit einem inneren und einem äußeren Kornbereich (core/shell), wobei die Halogenidzusammensetzung und/oder sonstige Modifizierungen, wie z.B. Dotierungen der einzelnen Kornbereiche unterschiedlich sind. Die mittlere Korngröße der Emulsionen liegt vorzugsweise zwischen 0,2 µm und 2,0 µm, die Korngrößenverteilung kann sowohl homo- als auch heterodispers sein. Die Emulsionen können außer dem Silberhalogenid auch organische Silbersalze enthalten, z.B. Silberbenztriazolat oder Silberbehenat.

Es können zwei oder mehrere Arten von Silberhalogenidemulsionen, die getrennt hergestellt werden, als Mischung verwendet werden.

Die fotografischen Emulsionen können nach verschiedenen Methoden (z.B. P. Glafkides. Chimie et Pysique Photographique, Paul Montel, Paris (1967), G.F. Duffin, Photographic Emulsion Chemistry, The Focal Press, London (1966), V.L. Zelikman et al, Making and Coating Photographic Emulsion, The Focal Press, London (1966)) aus löslichen Silbersalzen und löslichen Halogeniden hergestellt werden.

Als Bindemittel für die Silberhalogenide wird vorzugsweise Gelatine verwendet. Diese kann jedoch ganz oder teilweise durch andere synthetische, halbsynthetische oder auch natürlich vorkommende Polymere ersetzt werden.

Die Bindemittel sollen über eine ausreichende Menge an funktionellen Gruppen verfügen, so daß durch Umsetzung mit geeigneten Härtungsmitteln genügend widerstandsfähige Schichten erzeugt werden können. Solche funktionellen Gruppen sind insbesondere Aminogruppen, aber auch Carboxylgruppen, Hydroxylgruppen und aktive Methylengruppen.

Die fotografischen Emulsionen können Verbindungen zur Verhinderung der Schleierbildung oder zur Stabilisierung der fotografischen Funktion während der Produktion, der Lagerung oder der fotografischen Verarbeitung enthalten.

Besonders geeignet sind Azaindene, vorzugsweise Tetra- und Pentaazaindene, insbesondere solche, die mit Hydroxyl- oder Aminogruppen substituiert sind. Derartige Verbindungen sind z.B. von Birr. Z. Wiss. Phot. 47 (1952), S. 2-58 beschrieben worden. Weiter können als Antischleiermittel Salze von Metallen wie Quecksilber oder Cadmium, aromatische Sulfon- oder Sulfinsäuren wie Benzolsulfinsäure, oder stickstoffhaltige Heterocyclen wie Nitrobenzimidazol, Nitroindazol, (subst.) Benztriazole oder Benzthiazoliumsalze eingesetzt werden. Besonders geeignet sind Mercaptogruppen enthaltende Heterocyclen, z.B. Mercaptobenzthiazole, Mercaptobenzimidazole, Mercaptotetrazole, Mercaptothiadiazole, Mercaptopyrimidine, wobei diese Mercaptoazole auch eine wasserlöslichmachende Gruppe, z. B. eine Carboxylgruppe oder Sulfogruppe, enthalten können. Weitere geeignete Verbindungen sind in Research Disclosure Nr. 17643 (1978), Abschnitt VI, veröffentlicht.

Die Stabilisatoren können den Silberhalogenidemulsionen vor, während oder nach deren Reifung zugesetzt werden. Selbstverständlich kann man die Verbindungen auch anderen fotografischen Schichten, die einer Silberhalogenidschicht zugeordnet sind, zusetzen.

Es können auch Mischungen aus zwei oder mehreren der genannten Verbindungen eingesetzt werden.

Die Silberhalogenidemulsionen werden üblicherweise chemisch gereift, beispielsweise durch Einwirkung von Goldverbindungen oder Verbindungen des zweiwertigen Schwefels.

Die fotografischen Emulsionsschichten oder andere hydrophile Kolloidschichten des erfindungsgemäß hergestellten lichtempfindlichen Materials können oberflächenaktive Mittel für verschiedene Zwecke enthalten, wie Überzugshilfen, zur Verhinderung der elektrischen Aufladung, zur Verbesserung der Gleiteigenschaften, zum Emulgieren der Dispersion, zur Verhinderung der Adhäsion und zur Verbesserung der fotografischen Charakteristika (z.B. Entwicklungsbeschleunigung, hoher Kontrast, Sensibilisierung usw.).

Geeignete Sensibilisierungsfarbstoffe sind Cyaninfarbstoffe, insbesondere der folgenden Klassen:

1. Rotsensibilisatoren

Dicarbocyanine mit Naphthothiazol oder Benzthiazol als basischen Endgruppen, die in 5- und/oder 6-Stellung durch Halogen, Methyl, Methoxy substituiert sein können sowie 9.11-alkylen-verbrückte, insbesondere 9.11-Neopentylenthiadicarbocyanine mit Alkyl- oder Sulfoalkylsubstituenten am Stickstoff.

2. Grünsensibilisatoren

9-Ethyloxacarbocyanine, die in 5-Stellung durch Chlor oder Phenyl substituiert sind und am Stickstoff der Benzoxazolgruppen Alkyl- oder Sulfoalkylreste, vorzugsweise Sulfoalkylsubstituenten tragen.

3. Blausensibilisatoren

Methincyanine mit Benzoxazol, Benzthiazol, Benzselenazol, Naphthoxazol, Naphthothiazol als basischen Endgruppen, die in 5- und/oder 6-Stellung durch Halogen, Methyl, Methoxy substituiert sein können und mindestens einer vorzugsweise zwei, Sulfoalkylsubstituenten am Stickstoff tragen. Ferner Apomerocyanine mit einer Rhodaningruppe.

Auf Sensibilisatoren kann verzichtet werden, wenn für einen bestimmten Spektralbereich die Eigenempfindlichkeit des Silberhalogenids ausreichend ist, beispielsweise die Blauempfindlichkeit von Silberbromidiodiden.

Den unterschiedlich sensibilisierten Emulsionsschichten werden nicht diffundierende monomere oder polymere Farbkuppler zugeordnet, die sich in der gleichen Schicht oder in einer dazu benachbarten Schicht befinden können. Gewöhnlich werden den rotempfindlichen Schichten Blaugrünkuppler, den grünempfindlichen Schichten Purpurkuppler und den blauempfindlichen Schichten Gelbkuppler zugeordnet.

Farbkuppler zur Erzeugung des blaugrünen Teilfarbenbildes sind in der Regel Kuppler vom Phenol- oder $\alpha$-Naphtholtyp.

Farbkuppler zur Erzeugung des purpurnen Teilfarbenbildes sind in der Regel Kuppler vom Typ des Pyrazolazols, 5-Pyrazolons oder des Indazolons.

Farbkuppler zur Erzeugung des gelben Teilfarbenbildes sind in der Regel Kuppler mit einer offenkettigen Ketomethylengruppierung, insbesondere Kuppler vom Typ des $\alpha$-Acylacetamids; geeignete Beispiele hierfür sind $\alpha$-Benzoylacetanilidkuppler und $\alpha$-Pivaloylacetanilidkuppler. Letztere sind, wie bereits erwähnt, bevorzugt.

Bei den Farbkupplern kann es sich um 4-Äquivalentkuppler, aber auch um 2-Äquivalentkuppler handeln. Letztere leiten sich von den 4-Äquivalentkupplern dadurch ab, daß sie in der Kupplungsstelle einen Substituenten enthalten, der bei der Kupplung abgespalten wird.

Die Kuppler enthalten üblicherweise einen Ballastrest, um eine Diffusion innerhalb des Materials, d.h. sowohl innerhalb einer Schicht oder von Schicht zu Schicht, unmöglich zu machen. Anstelle von Kupplern mit einem Ballastrest können auch hochmolekulare Kuppler eingesetzt werden.

Geeignete Farbkuppler bzw. Literaturstellen, in denen solche beschrieben sind, finden sich in Research Disclosure 17 643 (1978), Kapitel VII.

Hochmolekulare Farbkuppler sind beispielsweise in DE-C-1 297 417, DE-A-24 07 569, DE-A-31 48 125, DE-A-32 17 200, DE-A-33 20 079, DE-A-33 24 932, DE-A-33 31 743, DE-A-33 40 376, EP-A-27 284, US-A-4 080 211 beschrieben. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Einarbeitung der Kuppler oder anderer Verbindungen in Silberhalogenidemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittels hängt von der jeweiligen Löslichkeit der Verbindung ab.

Methoden zum Einbringen von in Wasser im wesentlichen unlöslichen Verbindungen durch Mahlverfahren sind beispielsweise in DE-A-26 09 741 und DE-A-26 09 742 beschrieben.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 027, US-A-2 801 170, US-A-2 801 171 und EP-A-O 043 037 beschrieben.

Anstelle der hochsiedenden Lösungsmittel können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-O 130 115, US-A-4 291 113.

Die diffusionsfeste Einlagerung anionischer wasserlöslicher Verbindungen (z.B. von Farbstoffen) kann auch mit Hilfe von kationischen Polymeren, sogenannten Beizenpolymeren erfolgen.

Geeignete Ölbildner sind z.B. Phthalsäurealkylester, Phosphonsäureester, Phosphorsäureester, Citronensäureester, Benzoesäureester, Amide, Fettsäureester, Trimesinsäureester, Alkohole, Phenole, Anilinderivate, Kohlenwasserstoffe, Sulfone und Sulfoxide.

Beispiele für geeignete Ölbildner sind Dibutylphthalat, Dicyclohexylphthalat, Di-2-ethylhexylphthalat, Decylphthalat, Triphenylphosphat, Tricresylphosphat, 2-Ethylhexyldiphenylphosphat, Tricyclohexylphosphat, Tri-2-ethylhexylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-tert.-amylphenol, Dioctylacetat, Glycerintributyrat, Isostearyllactat, Trioctylcitrat, N,N-Dibutyl-2-butoxy-5-tert.-octylanilin, Paraffin, Dodecylbenzol und Diisopropylnaphthalin.

Das fotografische Material kann weiterhin UV-Licht absorbierende Verbindungen, Weißtöner, Abstandshalter, Filterfarbstoffe, Formalinfänger, Lichtschutzmittel, Antioxidantien, $D_{Min}$-Farbstoffe, Zusätze zur Verbesseder Farbstoff-, Kuppler- und Weißenstabilisierung sowie zur Verringerung des Farbschleiers, Weichmacher (Latices), Biocide und anderes enthalten. Darüber hinaus können Zwischenschichten sogenannte Weißkuppler und andere Verbindungen enthalten, die mit dem Entwickleroxidationsprodukt reagieren (Scavenger).

Die Schichten des fotografischen Materials können mit den üblichen Härtungsmitteln gehärtet werden. Geeignete Härtungsmittel sind z.B. Formaldehyd, Glutaraldehyd und ähnliche Aldehydverbindungen, Diacetyl, Cyclopentadion und ähnliche Ketonverbindungen, Bis-(2-chlorethylharnstoff), 2-Hydroxy-4,6-dichlor-1,3,5-triazin und andere Verbindungen, die reaktives Halogen enthalten (US-A-3 288 775, US-A-2 732 303, GB-A-974 723 und GB-A-1 167 207) Divinylsulfonverbindungen, 5-Acetyl-1,3-diacryloylhexahydro-1,3,5-triazin und andere Verbindungen, die eine reaktive Olefinbindung enthalten (US-A-3 635 718, US-A-3 232 763 und GB-A-994 869); N-Hydroxymethylphthalimid und andere N-Methylolverbindungen (US-A-2 732 316 und US-A-2 586 168); Isocyanate (US-A-3 103 437); Aziridinverbindungen (US-A-3 017 280 und US-A-2 983 611); Säurederivate (US-A-2 725 294 und US-A-2 725 295); Verbindungen vom Carbodiimidtyp (US-A-3 100 704); Carbamoylpyridiniumsalze (DE-A-22 25 230 und DE-A-24 39 551); Carbamoyloxypyridiniumverbindungen (DE-A-24 08 814); Verbindungen mit einer Phosphor-Halogen-Bindung (JP-A-113 929/83); N-Carbonyloximid-Verbindungen (JP-A-43353/81); N-Sulfonyloximido-Verbindungen (US-A-4 111 926), Dihydrochinolinverbindungen (US-A-4 4 013 468), 2-Sulfonyloxypyridiniumsalze (JP-A-110 762/81), Formamidiniumsalze (EP-A-0 162 308), Verbindungen mit zwei oder mehr N-Acyloximino-Gruppen (US-A-4 052 373), Epoxyverbindungen (US-A-3 091 537), Verbindungen von Isoxazoltyp (US-A-3 321 313 und US-A-3 543 292); Halogencarboxyaldehyde, wie Mucochlorsäure; Dioxanderivate, wie Dihydroxydioxan und Di-chlordioxan; und anorganische Härter, wie Chromalaun und Zirkonsulfat.

Die Härtung kann in bekannter Weise dadurch bewirkt werden, daß das Härtungsmittel der Gießlösung für die zu härtende Schicht zugesetzt wird, oder dadurch, daß die zu härtende Schicht mit einer Schicht überschichtet wird, die ein diffusionsfähiges Härtungsmittel enthält.

Unter den aufgeführten Klassen gibt es langsam wirkende und schnell wirkende Härtungsmittel sowie sogenannte Soforthärter, die besonders vorteilhaft sind. Unter Soforthärtern werden Verbindungen verstanden, die geeignete Bindemittel so vernetzen, daß unmittelbar nach Beguß, spätestens nach 24 Stunden, vorzugsweise spätestens nach 8 Stunden die Härtung so weit abgeschlossen ist, daß keine weitere durch die Vernetzungsreaktion bedingte Änderung der Sensitometrie und der Quellung des Schichtverbandes auftritt. Unter Quellung wird die Differenz von Naßschichtdicke und Trockenschichtdicke bei der wäßrigen Verarbeitung des Films verstanden (Photogr. Sci., Eng. 8 (1964), 275; Photogr. Sci. Eng. (1972), 449).

Bei diesen mit Gelatine sehr schnell reagierenden Härtungsmitteln handelt es sich z.B. um Carbamoylpyridiniumsalze, die mit freien Carboxylgruppen der Gelatine zu reagieren vermögen, so daß letztere mit freien Aminogruppen der Gelatine unter Ausbildung von Peptidbindungen und Vernetzung der Gelatine reagieren.

Es gibt diffusionsfähige Härtungsmittel, die auf alle Schichten innerhalb eines Schichtverbandes in gleicher Weise härtend wirken. Es gibt aber auch schichtbegrenzt wirkende, nicht diffundierende, niedermolekulare und hochmolekulare Härter. Mit ihnen kann man einzelne Schichten, z.B. die Schutzschicht besonders stark vernetzen. Dies ist wichtig, wenn man die Silberhalogenid-Schicht wegen der Silberdeckkrafterhöhung wenig härtet und mit der Schutzschicht die mechanischen Eigenschaften verbessern muß (EP-A 0 114 699).

Die erfindungsgemäßen farbfotografischen Materialien werden üblicherweise durch Entwickeln, Bleichen, Fixieren und Wässern oder Stabilisieren ohne nachfolgende Wässerung verareitet, wobei Bleichen und Fixieren zu einem Verarbeitungsschritt zusammengefaßt sein können. Als Farbentwicklerverbindung lassen sich sämtliche Entwicklerverbindungen verwenden, die die Fähigkeit besitzen, in Form ihres Oxidationsproduktes mit Farbkupplern zu Azomethin- bzw. Indophenolfarbstoffen zu reagieren. Geeignete Farbentwicklerverbindungen sind aromatische, mindestens eine

primäre Aminogruppe enthaltende Verbindungen vom p-Phenylendiamintyp, beispielsweise N,N-Dialkyl-p-phenylendiamine wie N,N-Diethyl-p-phenylendiamin, 1-(N-Ethyl-N-methansulfonamidoethyl)-3-methyl-p-phenylendiamin, N-Ethyl-N-3-hydroxypropyl-3-methyl-p-phenylendiamin und 1-(N-Ethyl-N-methoxyethyl)-3-methyl-p-phenylendiamin. Weitere brauchbare Farbentwickler sind beispielsweise in J. Amer. Chem. Soc. 73, 3106 (1951) und G. Haist, Modern Photographic Processing, 1979, John Wiley and Sons, New York, Seite 545 ff. beschrieben.

Nach der Farbentwicklung kann ein saures Stoppbad oder eine Wässerung folgen.

Üblicherweise wird das Material nach der Farbentwicklung gebleicht und fixiert. Als Bleichmittel können z.B. Fe (III)-Salze und Fe(III)-Komplexsalze wie Ferricyanide, Dichromate, wasserlösliche Kobaltkomplexe verwendet werden. Besonders bevorzugt sind Eisen-(III)-Komplexe von Aminopolycarbonsäuren, insbesondere z.B. von Ethylendiamintetraessigsäure, Propylendiamintetraessigsäure, Diethylentriaminpentaessigsäure, Nitrilotriessigsäure, Iminodiessigsäure, N-Hydroxyethyl-ethylendiamintriessigsäure, Alkyliminodicarbonsäuren und von entsprechenden Phosphonsäuren. Geeignete als Bleichmittel sind weiterhin Persulfate und Peroxide, z.B. Wasserstoffperoxid.

Auf das Bleichfixierbad oder Fixierbad folgt meist eine Wässerung, die als Gegenstromwäaserung ausgeführt ist oder aus mehreren Tanks mit eigener Wasserzufuhr besteht.

Günstige Ergebnisse können bei Verwendung eines darauf folgenden Schlußbades, das keinen oder nur wenig Formaldeyhd enthält, erhalten werden.

Die Wässerung kann aber durch ein Stabilisierbad vollständig ersetzt werden, das üblicherweise im Gegenstrom geführt wird. Dieses Stabilisierbad übernimmt bei Formaldehydzusatz auch die Funktion eines Schlußbades.

Beispiel 1

Auf ein beidseitig mit Polyethylen beschichtetes Papier wurden die folgenden beiden Schichten aufgebracht. Die Mengen beziehen sich jeweils auf ein $m^2$.

1. Schicht
Blauempfindliche Silberhalogenidemulsionsschicht aus 0,6 g $AgNO_3$
(99,5 Mol-% Chlorid, 0,5 Mol-% Bromid, mittlerer Korndurchmesser 0,78 µm)
2 g Gelatine
0,8 g Gelbkuppler Y-9
0,6 g TKP
0,3 g Stabilisator gemäß Tabelle 1

2. Schicht
2 g Gelatine
0,4 g Härtungsmittel der Formel

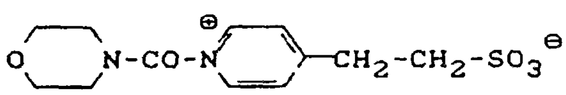

Die Proben wurden anschließend hinter einem graduierten Graukeil belichtet und mit den nachfolgend aufgeführten Verarbeitungsbädern in der üblichen Weise verarbeitet.

a) Farbentwickler - 45 s - 35°C

| | |
|---|---|
| Triethanolamin | 9,0 g/l |
| NN-Diethylhydroxyamin | 4,0 g/l |
| Diethylenglykol | 0,05 g/l |
| 3-Methyl-4-amino-N-ethyl-N-methansulfonaminoethyl-anilin-sulfat | 5,0 g/l |
| Kaliumsulfit | 0,2 g/l |
| Triethylenglykol | 0,05 g/l |
| Kaliumcarbonat | 22 g/l |
| Kaliumhydroxid | 0,4 g/l |
| Ethylendiamintetraessigsäure-di-Na-Salz | 2,2 g/l |
| Kaliumchlorid | 2,5 g/l |

(fortgesetzt)

| | |
|---|---|
| 1,2-Dihydroxybenzol-3,4,6-trisulfonsäure-trinatriumsalz<br>auffüllen mit Wasser auf 1000 ml; pH 10,0 | 0,3 g/l |

b) Bleichfixierbad - 45 s - 35°C

| | |
|---|---|
| Ammoniumthiosulfat | 75 g/l |
| Natriumhydrogensulfit | 13,5 g/l |
| Ammoniumacetat | 2,0 g/l |
| Ethylendiamintetraessigsäure (Eisen-Ammonium-Salz) | 57 g/l |
| Ammoniak 25 gew.-%ig | 9,5 g/l |
| Essigsäure | 9,0 g/l |
| auffüllen mit Wasser auf 1000 ml; pH 5,5 | |

c) Wässern - 2 min - 35°C

d) Trocknen

Die verarbeiteten Proben werden anschließend, abgedeckt mit einer UV-Schutzfolie, in einem Xenontestgerät zur Ermittlung der Lichtechtheit bestrahlt ($14.4 \cdot 10^6$ 1 x h).

Die UV-Schutzfolie ist wie folgt hergestellt worden: Auf einer mit einer Haftschicht versehenen transparenten Cellulosetriacetatfolie wurde eine Schicht aus 1,5 g Gelatine, 0,65 g UV-Absorber der folgenden Formel

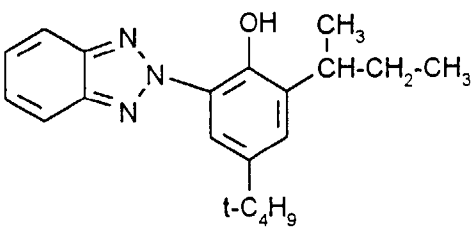

0,07 g Dioctylhydrochinon und 0,36 g Trikresylphosphat aufgetragen. Die Mengen beziehen sich auf 1 $m^2$.

Tabelle 1

| Probe | Stabilisator | Dichteabnahme in % bei Dichte | | | |
|---|---|---|---|---|---|
| | | | 0,5 | 1,0 | $D_{max}$ |
| 1 | Vergleich | - | 36 | 33 | 57 |
| 2 | Vergleich | V-1 | 22 | 18 | 45 |
| 3 | Vergleich | V-2 | 20 | 21 | 46 |
| 4 | Vergleich | V-3 | 22 | 20 | 50 |
| 5 | Vergleich | V-4 | 21 | 16 | 35 |
| 6 | Vergleich | V-5 | 23 | 17 | 33 |
| 7 | Erfindung | I-1 | 22 | 14 | 27 |

Wie Tabelle 1 zeigt, wird mit den erfindungsgemäßen Verbindungen besonders bei mittleren und hohen Dichten eine verbesserte Lichtstabilität der Gelbfarbstoffe erzielt.

V-1

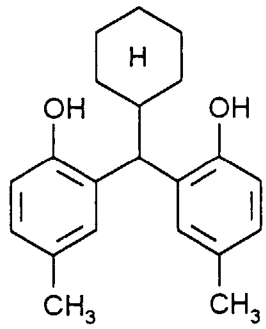

V-2

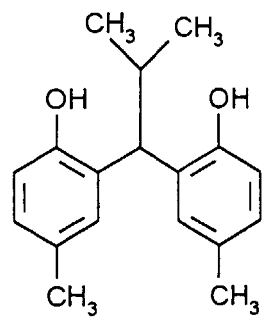

(JP 01/183.654)

V-3

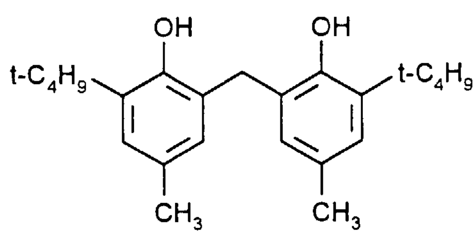

(US 3 700 455, Bsp. 1)

V-4

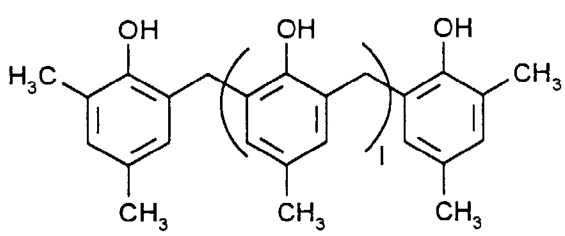

$\bar{M}_w = 950$

V-5

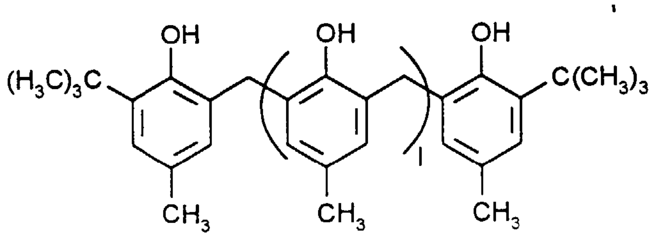

$\bar{M}_w = 1200$

Beispiel 2

Beispiel 1 wurde mit der Änderung wiederholt, daß anstelle des Gelbkupplers Y-9 der Gelbkuppler Y-21 in gleicher Menge verwendet wurde.

Tabelle 2

| Probe | Stabilisator | Dichteabnahme in % bei Dichte | | | |
|---|---|---|---|---|---|
| | | | 0,5 | 1,0 | $D_{max}$ |
| 1 | Vergleich | - | 16 | 15 | 39 |
| 2 | Vergleich | V-1 | 17 | 15 | 34 |
| 3 | Vergleich | V-2 | 16 | 13 | 32 |
| 4 | Vergleich | V-3 | 17 | 16 | 35 |
| 5 | Vergleich | V-4 | 17 | 12 | 18 |
| 6 | Vergleich | V-5 | 16 | 10 | 16 |
| 7 | Erfindung | I-1 | 18 | 7 | 12 |

Mit den erfindungsgemäßen Verbindungen als Lichtschutzmittel wird die Lichtstabilität des Gelbfarbstoffes bei Dichte 1 und in den hohen Dichten signifikant verbessert.

**Patentansprüche**

**1.** Farbfotografisches Material, das auf einem Träger wenigstens eine blauempfindliche, wenigstens einen Gelbkuppler enthaltende Silberhalogenidemulsionsschicht, wenigstens eine grünempfindliche, wenigstens einen Purpurkuppler enthaltende Silberhalogenidemulsionsschicht, wenigstens eine rotempfindliche, wenigstens einen Blaugrünkuppler enthaltende Silberhalogenidemulsionsschicht sowie übliche nicht-lichtempfindliche Schichten enthält, dadurch gekennzeichnet, daß wenigstens eine blauempfindliche, einen Gelbkuppler enthaltende Schicht eine Verbindung der Formel (I)

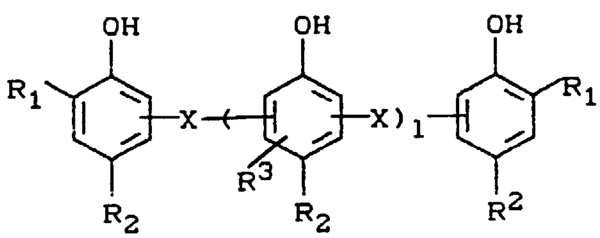

(I)

enthält, worin

$R_1$ Wasserstoff oder Alkyl,

$R_2$ Wasserstoff oder Alkyl,

$R_3$ Wasserstoff oder Alkyl,

1 1 bis 20, vorzugsweise 2 bis 8,

X Cycloalkylen,

$R_4$ Wasserstoff oder Alkyl,

$R_5$ Wasserstoff oder Alkyl und

n 1 bis 6, vorzugsweise 1 bis 4 bedeuten.

2. Farbfotografisches Material nach Anspruch 1, dadurch gekennzeichnet, daß die Verbindung der Formel I in der wenigstens einen blauempfindlichen, gelbkuppelnden Schicht in einer Menge von 0,1 bis 2 Mol/Mol Kuppler eingesetzt wird.

3. Farbfotografisches Silberhalogenidmaterial nach Anspruch 1, dadurch gekennzeichnet, daß der Gelbkuppler ein 2-Äquivalentpivaloylkuppler ist, dessen Abgangsgruppe entweder über Sauerstoff oder über Stickstoff mit der kuppelnden Position verbunden ist.

4. Farbfotografisches Silberhalogenidmaterial nach Anspruch 1, dadurch gekennzeichnet, daß die Silberhalogenidemulsionen zu wenigstens 80 Mol-% AgCl-Emulsionen sind.

5. Farbfotografisches Silberhalogenidmaterial nach Anspruch 1, dadurch gekennzeichnet, daß die Reste $R_1$ untereinander gleich und die Reste $R_2$ untereinander gleich sind und Wasserstoff oder $C_1$-$C_{12}$-Alkyl bedeuten.

6. Farbfotografisches Silberhalogenidmaterial nach Anspruch 1, dadurch gekennzeichnet, daß X

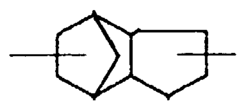

bedeutet.

7. Farbfotografisches Silberhalogenidmaterial nach Anspruch 1, dadurch gekennzeichnet, daß

$R_3$ Wasserstoff und
$R_2$ tert.-Butyl ist.

**Claims**

1. Colour photographic material which contains on a support at least one blue-sensitive silver halide emulsion layer containing at least one yellow coupler, at least one green-sensitive silver halide emulsion layer containing at least one magenta coupler, at least one red-sensitive silver halide emulsion layer containing at least one cyan coupler, together with conventional non-photosensitive layers, characterised in that at least one blue-sensitive layer containing a yellow coupler contains a compound of the formula (I)

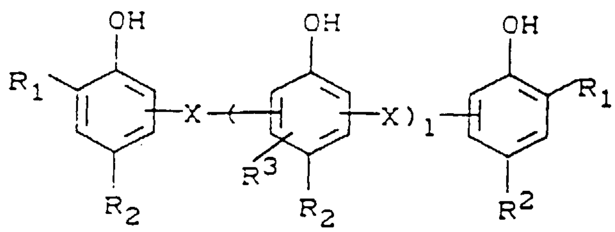

(I)

in which

$R_1$ means hydrogen or alkyl,

$R_2$ means hydrogen or alkyl,

$R_3$ means hydrogen or alkyl,

1 means 1 to 20, preferably 2 to 8,

X means cycloalkylene,

$R_4$ means hydrogen or alkyl,

$R_5$ means hydrogen or alkyl and

n means 1 to 6, preferably 1 to 4.

**2.** Colour photographic material according to claim 1, characterised in that the compound of the formula I is used in the yellow-coupling blue-sensitive layer, of which there is at least one, in a quantity of 0.1 to 2 mol/mol of coupler.

**3.** Colour photographic silver halide material according to claim 1, characterised in that the yellow coupler is a 2-equivalent pivaloyl coupler, the leaving group of which is attached to the coupling position either with oxygen or with nitrogen.

**4.** Colour photographic silver halide material according to claim 1, characterised in that the silver halide emulsions are at least 80 mol% AgCl emulsions.

**5.** Colour photographic silver halide material according to claim 1, characterised in that the residues $R_1$ are identical to each other and the residues $R_2$ are identical to each other and mean hydrogen or $C_1$-$C_{12}$ alkyl.

**6.** Colour photographic silver halide material according to claim 1, characterised in that X means X

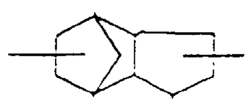

**7.** Colour photographic silver halide material according to claim 1, characterised in that

$R_3$ is hydrogen
$R_2$ is tert.-butyl.

**Revendications**

**1.** Matériau photographique couleur, qui contient sur un support au moins une couche d'émulsion d'halogénure d'argent sensible au bleu contenant au moins un coupleur pour jaune, au moins une couche d'émulsion à l'halogénure d'argent sensible au vert contenant au moins un coupleur pour magenta, au moins une couche d'émulsion d'halogénure d'argent sensible au rouge contenant au moins un coupleur pour cyan ainsi que les couches usuelles non sensibles à la lumière, caractérisé en ce qu'au moins une couche sensible au bleu contenant un coupleur pour jaune contient un composé de formule (I)

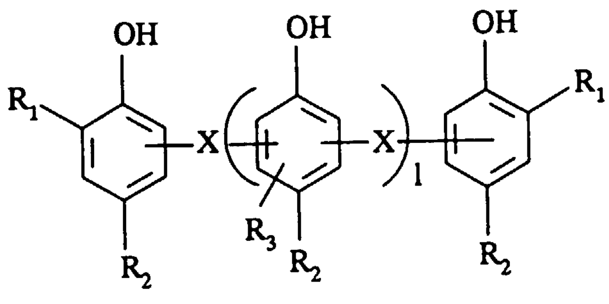

(I)

où

$R_1$ représente l'hydrogène ou un alkyle,
$R_2$ est un hydrogène ou un alkyle,
$R_3$ est un hydrogène ou un alkyle,
1 est 1 à 20, de préférence 2 à 8,

X est un cycloalkylène,
$R_4$ est un hydrogène ou un alkyle,
$R_5$ est un hydrogène ou un alkyle et
n est 1 à 6, de préférence 1 à 4.

2. Matériau photographique couleur selon la revendication 1, caractérisé en ce que le composé de formule I est utilisé dans au moins une couche sensible au bleu pour former le jaune par copulation en une quantité de 0,1 à 2 mol/mol de coupleur.

3. Matériau à l'halogénure d'argent photographique couleur selon la revendication 1, caractérisé en ce que le coupleur pour jaune est un coupleur pivaloyle à 2 équivalents, dont le groupe labile est lié soit par de l'oxygène, soit par de l'azote à la position de couplage.

4. Matériau à l'halogénure d'argent photographique couleur selon la revendication 1, caractérisé en ce que les émulsions d'halogénure d'argent sont pour au moins 80 % molaire des émulsions d'AgCl.

5. Matériau à l'halogénure d'argent photographique couleur selon la revendication 1, caractérisé en ce que les restes $R_1$ sont identiques entre eux et les restes $R_2$ sont identiques entre eux et représentent un hydrogène ou un alkyle $C_1$-$C_{12}$.

6. Matériau à l'halogénure d'argent photographique couleur selon la revendication 1, caractérisé en ce que X est

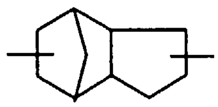

7. Matériau à l'halogénure d'argent photographique couleur selon la revendication 1, caractérisé en ce que

$R_3$ est un hydrogène et
$R_2$ est un tert.-butyle.